# FASCICULE DE BREVET EUROPEEN

(11) **EP 1 257 308 B1**
(45) Date de publication et mention de la délivrance du brevet: **08.06.2005**
(21) Numéro de dépôt: 01903875.1
(22) Date de dépôt: 05.01.2001
(51) Int. Cl.: A61M 5/31, A61M 5/32, A61M 5/50

(54) **DISPOSITIF D'INJECTION D'UNE DOSE DE LIQUIDE NOTAMMENT MEDICAMENTEUX**
INJEKTIONSVORRICHTUNG ZUR VERABREICHUNG VON INSBESONDERE MEDIZINISCHEN FLÜSSIGKEITEN
DEVICE FOR INJECTING A DOSE OF LIQUID, IN PARTICULAR MEDICINAL LIQUID

(30) Priorité: 07.01.2000 FR 0000192; 07.01.2000 FR 0000193
(43) Date de publication de la demande: 20.11.2002
(73) Titulaire: Glaxo Group Limited, Greenford Middlesex UB6 0NN (GB)
(72) Inventeur: LOURENCO, Armindo, F-62950 Noyelles-Godault (FR); STEIMER, Benoît, F-67000 Strasbourg (FR)
(74) Mandataire: Sewell, Richard Charles
(86) Numéro de dépôt international: PCT/FR2001/000031
(87) Numéro de publication internationale: WO 2001/049347

(56) Documents cités:
- EP-A- 0 471 335
- WO-A-99/17823
- DE-U- 9 117 140
- DE-U- 29 515 752
- FR-A- 2 390 966
- FR-A- 2 784 033
- US-A- 4 929 232
- US-A- 4 973 318

## Description

L'invention concerne un dispositif d'injection d'une dose de liquide notamment médicamenteux.

A l'heure actuelle, la plupart des dispositifs d'injection sont, à des fins d'hygiène et de sécurité, des dispositifs à usage unique et comprennent, outre une seringue classique ou non, des éléments de protection conçus pour masquer l'aiguille après utilisation et interdire toute réutilisation ultérieure du dispositif d'injection.

De tels dispositifs d'injection sont donc constitués d'une pluralité d'éléments dont un élément doté d'organes appui-doigts qui sont acheminés généralement entre deux rails parallèles et distribués vers des machines d'assemblage conçues pour les solidariser en translation.

De plus, en règle générale, les divers éléments des dispositifs d'injection sont dotés d'organes de blocage en rotation permettant de les verrouiller en rotation les uns par rapport aux autres. A cet effet, les chaînes d'assemblage sont donc pourvues de moyens spécifiques de repérage de la position angulaire des éléments et notamment de l'élément doté d'organes appui-doigts et d'indexation de cette position angulaire en vue de permettre l'assemblage.

Par ailleurs, une fois l'assemblage réalisé, et afin de garantir la non-utilisation préalable des dispositifs d'injection, deux solutions existent à l'heure actuelle.

La première solution consiste à conditionner ces dispositifs d'injections dans un emballage stérile du type pochette, blister ...

La seconde solution consiste à équiper ces dispositifs d'injection d'un capuchon de protection amovible apte à coiffer la portion postérieure de l'élément doté d'appui-doigts, de façon à venir en butée contre lesdits organes appui-doigts. Selon cette solution, en outre, le capuchon de protection et l'embout classique protège-aiguille sont solidarisés par des sceaux de sécurité, tels que des points de soudure, visant à permettre de vérifier et de garantir le non-retrait préalable de l'un quelconque de ces deux éléments.

L'intérêt de cette seconde solution réside dans le fait que "l'inviolabilité" du produit est obtenue au moyen d'un simple capuchon de protection qui, d'une part, peut-être mis en place directement sur la chaîne d'assemblage sans nécessiter une quelconque opération de conditionnement ultérieure, et d'autre part, restreint l'encombrement des produits finis par rapport à ceux obtenus selon la première solution.

L'inconvénient majeur de cette seconde solution réside par contre dans le fait que "l'inviolabilité" nécessite de solidariser le capuchon de protection, ce qui dans la pratique impose d'exercer un effort relativement important en vue du retrait de ce capuchon de protection.

La présente invention vise en premier lieu à simplifier les chaînes d'assemblage et a pour premier objectif de fournir un dispositif d'injection dont l'indexation en rotation de l'élément doté d'organes appui-doigts est obtenue de façon automatique sans nécessiter de moyens spécifiques.

Un autre objectif de l'invention est de fournir un dispositif d'injection dont l'opération d'assemblage de l'élément doté d'organes appui-doigts avec un autre élément est obtenue de façon très simple.

Un autre objectif de l'invention est de fournir un dispositif d'injection comprenant un capuchon de protection assurant "l'inviolabilité" dudit dispositif d'injection et dont le retrait est obtenu de façon très aisée.

A cet effet, l'invention vise en premier lieu un dispositif d'injection selon la revendication 1.

La forme spécifique de la collerette appui-doigts du dispositif d'injection selon l'invention conduit avantageusement, lorsque l'élément doté de cette collerette est véhiculé entre deux rails parallèles, à amener cet élément à pivoter automatiquement sur lui-même jusqu'à l'obtention d'une position angulaire de transport stable dans laquelle les portions de préhension reposent sur lesdits rails.

De ce fait, quelle que soit la position angulaire initiale dans laquelle l'élément doté de la collerette est déposé entre les rails, l'orientation finale obtenue est toujours sensiblement la même de sorte que l'indexation en rotation de cet élément ne nécessite ni dispositif de repérage ni dispositif d'indexation.

Il est à noter, en outre, que la précision quant à l'orientation finale obtenue est fonction de la longueur de la base supérieure des profils de forme convexe. En effet, si cette base supérieure présente une longueur supérieure ou égale au diamètre de l'élément tubulaire, l'orientation finale obtenue est strictement toujours la même. Par contre si cette longueur est inférieure à ce diamètre l'orientation obtenue peut varier de quelques degrés fonctions de la différence de longueur.

De façon avantageuse, chaque portion d'indexation de la collerette comporte une face inférieure présentant un profil courbe de forme arrondie convexe.

De plus, selon un mode de réalisation avantageux, chaque portion d'indexation de la collerette présente la forme d'une ondulation définissant une échancrure de forme concave.

De telles échancrures permettent notamment, tel qu'on le comprendra mieux plus loin, de garantir le maintien de la position d'indexation de l'élément doté de la collerette appui-doigts lors de l'assemblage de ce dernier avec un autre élément au niveau d'une machine d'assemblage. En effet, le maintien de cette position d'indexation peut-être assuré de façon très aisée en utilisant un outil tubulaire apte à coiffer l'élément doté de la collerette, comportant un tronçon d'extrémité de forme conjuguée de celle de la face supérieure de la collerette, et présentant donc deux portions de profil convexe de forme conjuguée de la forme concave des échancrures. De plus, cet outil tubulaire conduit à corriger d'éventuels défauts d'orientation, dans le cas notamment où la longueur de la base supérieure des échancrures est inférieure au diamètre de l'élément tubulaire.

Selon un mode de réalisation avantageux, le dispositif d'injection comprend un capuchon de protection apte à coiffer le tronçon postérieur de l'élément tubulaire du corps de seringue de façon à venir en butée contre la collerette.

Dans ce cas et de façon avantageuse, selon l'invention, le capuchon de protection comporte une paroi périphérique dotée d'un tronçon d'extrémité comprenant, pour chaque portion d'indexation de la collerette appui-doigts, une portion de paroi périphérique, dite d'assemblage, présentant un profil courbe de forme arrondie convexe complémentaire de la face supérieure de ladite portion d'indexation, adaptée pour s'emboîter dans cette dernière.

Ce capuchon de protection et les organes appui-doigts du corps de seringue possèdent ainsi des portions d'assemblage conduisant en premier lieu à l'obtention d'une force de serrage du fait des formes complémentaires convexe et concave des faces en contact, et permettant d'assurer "l'inviolabilité" au moyen d'un éventuel sceau de faible résistance et donc facilement cassable.

De plus, le retrait du capuchon de protection est obtenu en faisant tourner ce dernier dans l'un ou l'autre sens de rotation, rotation au cours de laquelle les profils des portions d'assemblage dudit capuchon et des organes appui-doigts conduisent à entraîner un déplacement axial de ce capuchon, de sorte que le retrait de ce dernier s'assimile à une simple opération de dévissage ne nécessitant pas d'exercer un effort notoire.

Il est à noter, en outre, que la présence de deux portions d'assemblage symétriques conduit, d'une part, à augmenter l'effort de serrage obtenu, et d'autre part, à faciliter le retrait du capuchon de protection du fait que le déplacement de ce dernier résulte d'un effort dont la résultante est parfaitement axiale.

Par ailleurs, de façon avantageuse, le capuchon de protection comporte au moins un organe interne de blocage en translation en saillie par rapport à la face interne de la paroi périphérique dudit capuchon, l'élément tubulaire du corps de seringue comportant une paroi périphérique dont la face externe présente, en position conjuguée de chacun desdits organes de blocage en translation, un évidemment apte à loger ce dernier.

De tels organes de blocage en translation permettent de réduire à sa plus simple expression la résistance du sceau de sécurité visant à garantir "l'inviolabilité" du dispositif d'injection, et voire même de supprimer ce dernier.

En outre, de façon avantageuse, les organes de blocage en translation du capuchon de protection consistent en deux ergots internes diamétralement opposés, chaque évidement du corps de seringue consistant en une lumière apte à loger un desdits ergots.

D'autres caractéristiques, buts et avantages de l'invention ressortiront de la description détaillée qui suit en référence aux dessins annexés qui en représentent à titre d'exemple non limitatif un mode de réalisation préférentiel. Sur ces dessins :
- la figure 1 est une coupe longitudinale par un plan axial d'un dispositif d'injection conforme à l'invention, représenté avant utilisation,
- la figure 2 est une vue en perspective de ce dispositif d'injection,
- la figure 3 est une vue en perspective d'un premier élément, consistant en un étui protecteur, de ce dispositif d'injection,
- la figure 4 est une coupe longitudinale par un plan axial A de cet étui protecteur,
- la figure 5 est une coupe longitudinale par un plan axial B de cet étui protecteur,
- la figure 6 est une coupe transversale par un plan C de cet étui protecteur,
- la figure 7 est une coupe transversale par un plan D de cet étui protecteur,

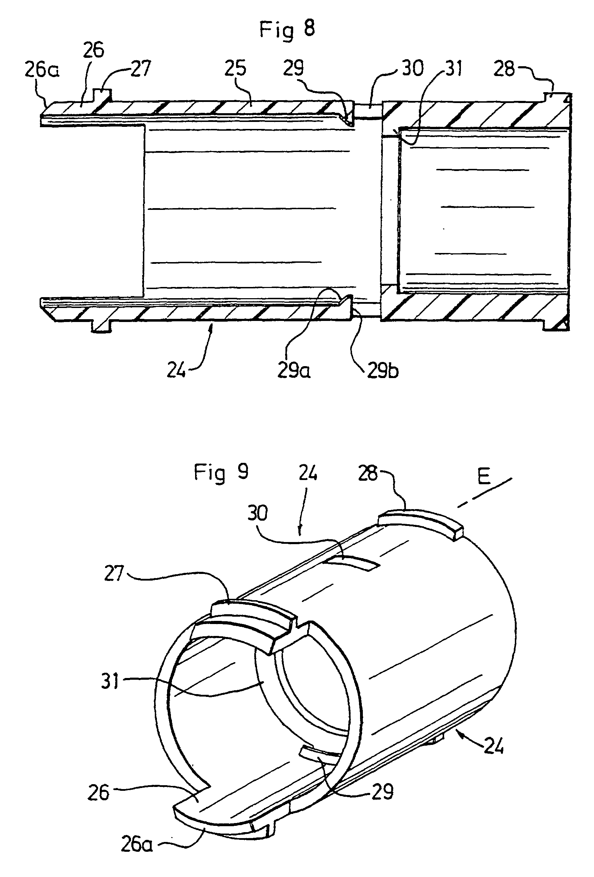

- la figure 8 est une coupe longitudinale par un plan axial E d'un deuxième élément, consistant en une bague de verrouillage, de ce dispositif d'injection,
- la figure 9 est une vue en perspective de cette bague de verrouillage,
- la figure 10 est une coupe longitudinale par un plan axial F d'un troisième élément, consistant en un embout à griffes, de ce dispositif d'injection,
- la figure 11 est une vue en perspective de cet embout à griffes,
- la figure 12 est une vue en perspective schématique d'une unité de transfert de l'élément de l'étui protecteur doté d'organes appui-doigts,
- et la figure 13 est une vue schématique partiellement en coupe d'une machine d'assemblage des deux éléments de l'étui protecteur.

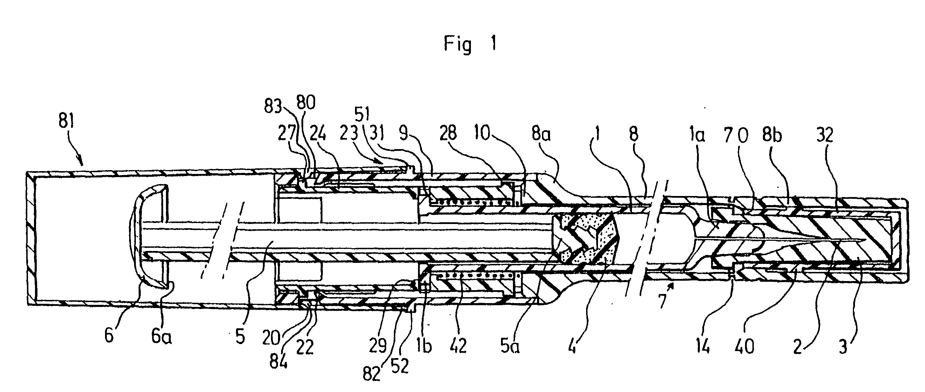

Le dispositif d'injection selon l'invention représenté à la figure 1 comprend, en premier lieu, une seringue pré-remplie 1 de type traditionnel, telle que par exemple réalisée en verre, comportant de façon classique un nez antérieur 1a sur lequel est montée une aiguille 2, et une collerette 1b au niveau de son extrémité postérieure.

Cette seringue 1 comporte également de façon classique un embout 3 protège-aiguille 2 adapté pour être monté de façon étanche sur le nez antérieur 1a de ladite seringue.

Elle comprend également de façon classique un piston 4 délimitant la chambre remplie d'une dose de liquide, percé d'un alésage borgne taraudé dans lequel vient se visser l'extrémité filetée 5a d'une tige de piston 5 dotée au niveau de son extrémité opposée d'un poussoir 6.

Ce poussoir 6 se présente sous la forme d'une coupelle présentant une tranche 6a à profil oblique à des fins explicitées plus loin.

Le dispositif d'injection selon l'invention comprend également un ensemble de protection de la seringue 1 adapté pour être entièrement pré-assemblé avant que ladite seringue initialement pré-remplie soit mise en place dans ledit ensemble de protection.

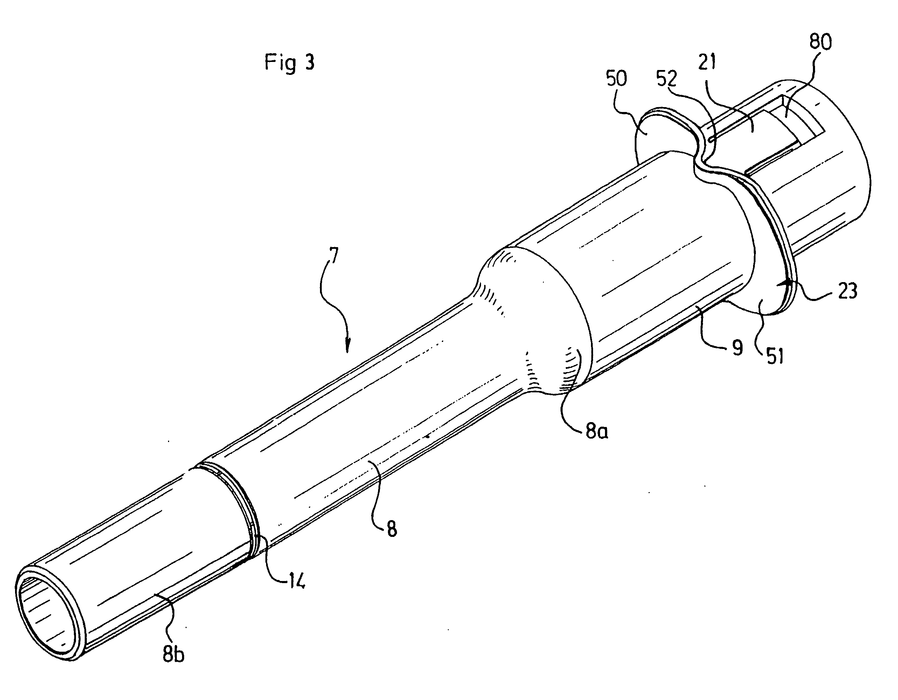

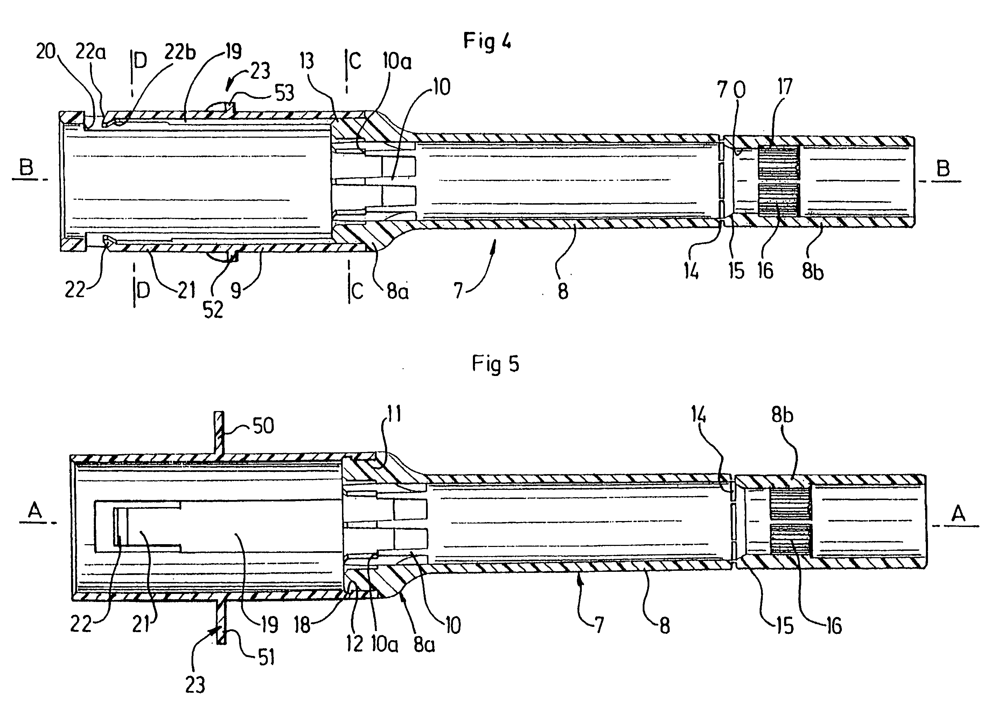

Cet ensemble de protection comprend en premier lieu un étui protecteur 7 représenté aux figures 3 à 7 composé de deux corps tubulaires antérieur 8 et postérieur 9 adaptés pour venir s'emmancher dans le prolongement l'un de l'autre.

Le corps antérieur 8 présente un diamètre interne conjugué du diamètre externe de la seringue 1 et une longueur adaptée pour loger l'aiguille 2 dotée de son embout protège-aiguille 3 et sensiblement 80% de la longueur de la seringue 1.

Au niveau de son extrémité postérieure, ce corps antérieur 8 comporte un tronçon postérieur 8a de forme externe sensiblement ovoïde de diamètres externes supérieurs au diamètre externe courant dudit corps antérieur, évidé intérieurement de façon à comporter des nervures longitudinales internes telles que 10 définissant un diamètre interne identique à celui du diamètre interne courant de ce corps antérieur 8.

Chacune de ces nervures 10 présente, en outre, un épaulement 10a délimitant une portion d'extrémité postérieure de diamètre interne sensiblement supérieur au diamètre de la seringue 1.

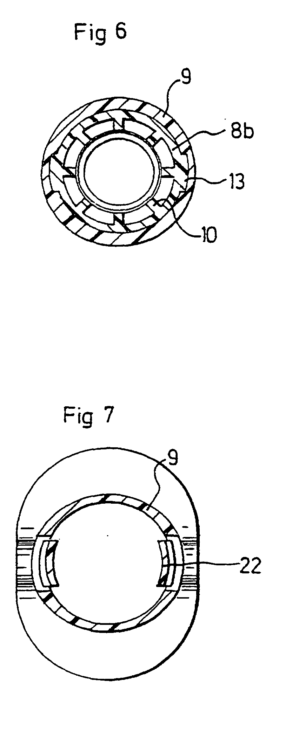

Côté externe, ce tronçon postérieur 8a comporte une gorge annulaire 11 délimitée par un jonc postérieur 12 de clipsage. De plus, tel que représenté à la figure 6, cette gorge 11 est interrompue par deux ergots tels que 13 diamétralement opposés faisant saillie radialement par rapport au jonc de clipsage 12.

Au niveau de son extrémité antérieure, le corps antérieur 8 comporte un tronçon antérieur 8b sécable délimité par une zone annulaire frangible 14 positionnée de façon à se trouver sensiblement au niveau du nez antérieur 1a de la seringue 1 une fois celle-ci mise en place.

Ce tronçon sécable 8b présente intérieurement au niveau de son extrémité postérieure un profil oblique 15 formant une rampe d'accès à une portion postérieure constituant un col 70 de diamètre sensiblement inférieur à celui du diamètre interne courant du corps antérieur 8.

A l'avant de ce col 70, le tronçon sécable 8b comporte, en outre, une pluralité de crans longitudinaux tels que 16 répartis sur la périphérie de la face interne dudit tronçon sécable et délimitant un diamètre interne identique à celui dudit col, de sorte que les rainures entre lesdits crans définissent un épaulement 17 avec l'extrémité antérieure de ce col 70.

En dernier lieu, concernant ce tronçon sécable 8b, la portion antérieure de ce dernier située à l'avant des crans 16 présente un diamètre interne identique au diamètre séparant le fond des crans.

Le corps postérieur 9 présente quant à lui une forme sensiblement ovoïde conjuguée de celle du tronçon postérieur 8a du corps antérieur 8, adaptée pour venir s'emmancher sur ledit tronçon postérieur 8a.

A cet effet, ce corps postérieur 9 comporte, en premier lieu, une gorge annulaire interne 18 agencée pour loger le jonc de clipsage 12. Il comporte, en outre, deux rainures longitudinales internes telles que 19 diamétralement opposées, aptes à loger chacune un ergot 13, de façon à assurer un blocage en rotation des deux corps 8, 9, lesdites rainures étant interrompues à faible distance de l'extrémité postérieure de ce corps postérieur 9, de façon que ce dernier présente un épaulement interne 20 au droit de l'extrémité de ces rainures 19.

Tel que représenté à la figure 6, les rainures 19 sont ménagées selon le diamètre le plus grand du corps postérieur 9, de façon à minimiser l'épaisseur de la paroi dudit corps postérieur.

Le corps postérieur 9 présente, en outre, deux languettes déformables telles que 21 ménagées chacune dans une rainure 19 au niveau de l'extrémité postérieure de cette dernière. Chacune de ces languettes 21 est formée par une découpe en forme de U ménagée dans la paroi dudit corps postérieur, et une lumière telle que 80, permettant notamment le démoulage, est ménagée dans ladite paroi, en amont de chaque languette 21.

Chacune de ces languettes 21 comporte au niveau de son extrémité postérieure un crochet transversal 22 faisant saillie à l'intérieur du corps postérieur 9. Chacun de ces crochets comporte une face postérieure anti-retour 22a sensiblement radiale, et une face antérieure oblique 22b formant une rampe.

En dernier lieu, le corps postérieur 9 comporte une collerette externe 23 d'appui digital.

Selon l'invention, cette collerette 23 de forme générale annulaire comporte :
- deux portions de contour 50, 51 diamétralement opposées, sensiblement planes définissant deux faces d'appui pour les doigts de l'utilisateur,
- et, entre les faces d'appui 50, 51, deux ondulations 52, 53, diamétralement opposées définissant deux échancrures courbes de forme concave.

L'ensemble de protection comprend, en deuxième lieu, une bague de verrouillage 24 de forme adaptée pour venir s'insérer dans le corps postérieur 9, en étant présentée en regard de la face antérieure de ce dernier.

Cette bague de verrouillage 24 de longueur adaptée pour s'insérer dans le corps postérieur 9 se présente sous la forme d'un manchon cylindrique 25 prolongé postérieurement par deux pattes telles que 26 diamétralement opposées en forme de secteur cylindrique.

Chacune de ces deux pattes 26 présente, en premier lieu, une face d'extrémité postérieure 26a à profil oblique complémentaire de celui de la tranche 6a du poussoir 6 de la tige de piston 5.

Chacune de ces pattes 26 comporte, en outre, sensiblement à mi-longueur, une nervure externe transversale 27, adaptée pour pouvoir coulisser dans une des rainures 19 du corps postérieur 9.

Le manchon cylindrique 25 de cette bague de verrouillage 24 comporte, quant à lui, axées sur les mêmes génératrices que les nervures transversales 27, deux nervures transversales telles que 28 également adaptées pour coulisser dans les rainures 19 du corps postérieur 9, et ménagées au niveau de l'extrémité antérieure dudit manchon.

Ce manchon cylindrique 25 comprend également sensiblement à mi-longueur et dans l'alignement axial des nervures 27, 28 précitées, deux nervures internes telles que 29 diamétralement opposées en aval de chacune desquelles la paroi périphérique dudit manchon est percée d'une lumière telle que 30 permettant le démoulage de la contre-dépouille.

Chacune de ces nervures internes 29 présente une face postérieure 29a à profil oblique formant rampe et une face antérieure 29b radiale de blocage anti-retour.

Enfin, le manchon cylindrique 25 comprend en aval des lumières 30 et à une distance des nervures internes 29 conjuguée de l'épaisseur de la collerette 1b de la seringue 1, un épaulement interne annulaire 31.

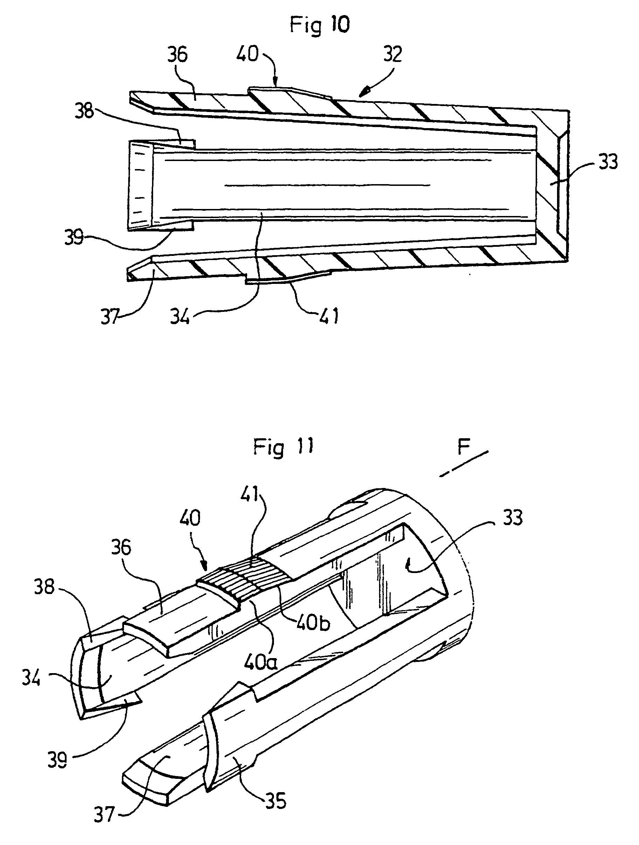

Le dispositif d'injection selon l'invention comprend, en outre, un embout à griffes 32 représenté aux figures 10 et 11 destiné à venir coiffer l'embout protège-aiguille 3 et à entraîner le retrait de ce dernier après rupture de la zone frangible 14 du corps antérieur 8 de l'étui protecteur 7.

Cet embout à griffes 32 présente une paroi frontale cylindrique 33, de diamètre adapté pour pénétrer dans le tronçon sécable 8b, en périphérie de laquelle s'étendent sensiblement orthogonalement quatre pattes longitudinales distinctes 34, 35, 36, 37 uniformément réparties par rapport à l'axe de ladite paroi :
- deux pattes 34, 35 diamétralement opposées dotées au niveau de leur extrémité libre chacune de deux griffes latérales telles que 38, 39 adaptées pour pouvoir s'ancrer dans l'embout protège-aiguille 3,
- deux autres pattes 36, 37 diamétralement opposées présentant, en position intermédiaire de leur longueur, chacune un bossage externe tel que 40 présentant une face externe pourvue de crans longitudinaux tels que 41 conjugués des crans 16 du tronçon sécable 8b. De plus, chaque bossage 40 présente une portion postérieure cylindrique 40a précédée d'une portion antérieure inclinée 40b formant rampe.

Il est à noter, en outre, que tel que représenté à la figure 10, lors du moulage, les pattes 34-37 sont réalisées en position sensiblement "ouverte", c'est-à-dire inclinées vers l'extérieur par rapport à l'axe de la paroi frontale 33.

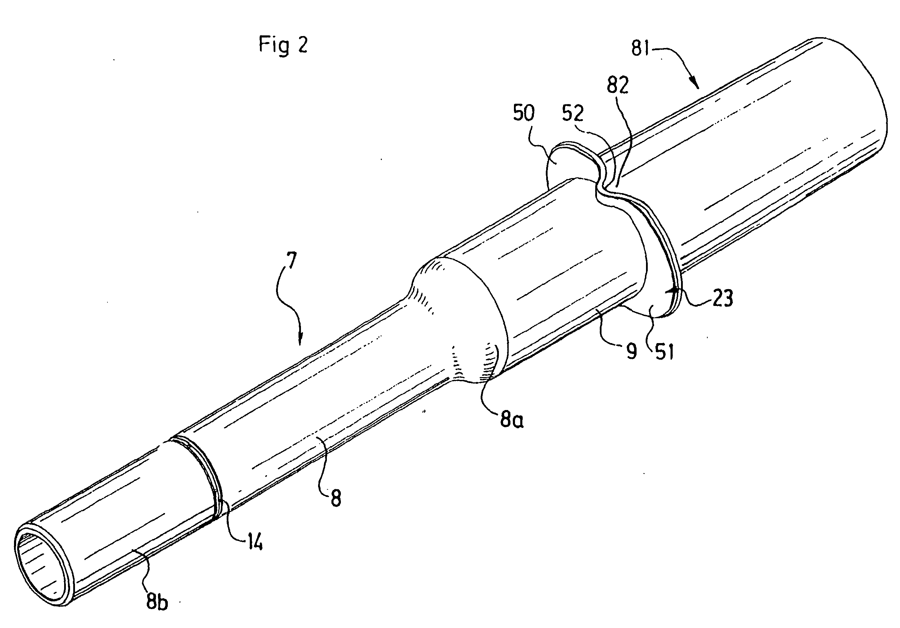

Tel que représenté aux figures 1 et 2, le dispositif d'injection comporte, enfin, un capuchon 81 de protection apte à coiffer le poussoir 6 et les extrémités postérieures de la tige de piston 5 et de l'étui protecteur 7, de façon à venir en butée contre la collerette 23.

Ce capuchon de protection 81 comprend un bord inférieur présentant un profil complémentaire de celui de la collerette 23, et comportant donc deux oreilles telles que 82 diamétralement opposées de forme convexe conjuguée de la forme concave des ondulations 52, 53 de ladite collerette.

Les formes spécifiques de la collerette 23 et du capuchon de protection 81 permettent en premier lieu d'obtenir un effort de serrage assurant un bon maintien en place dudit capuchon de protection. De plus, elles permettent de retirer aisément ce capuchon de protection 81 en imprimant à ce dernier un mouvement de rotation qui provoque un déplacement axial dudit capuchon de protection du fait des profils concaves et convexes conjugués des échancrures 52, 53 et oreilles 82.

Par ailleurs, le capuchon de protection 81 comporte deux ergots internes 83, 84 diamétralement opposés ménagés de façon à venir se loger chacun dans une lumière 80 de l'étui de protection 7 lors de la mise en place dudit capuchon de protection. De tels ergots 83, 84 et lumières 80 forment un système de verrouillage qui permet, si ce n'est de supprimer les sceaux de sécurité, d'au moins notablement diminuer la résistance de ces derniers.

Les étapes d'assemblage de ce dispositif d'injection sont décrites ci-dessous.

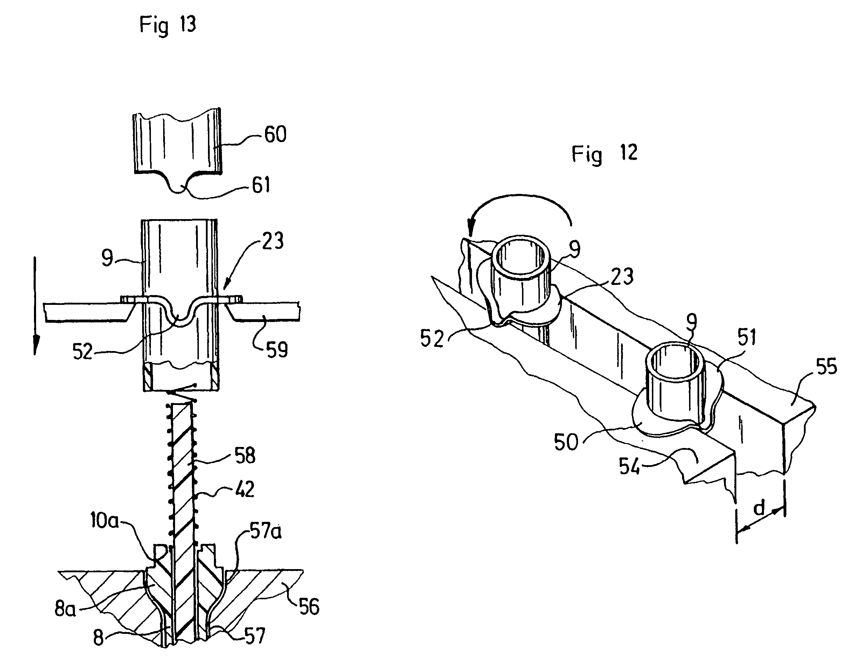

En premier lieu, tel que représenté à la figure 12, le corps postérieur 9 de l'étui protecteur 7 est acheminé entre deux rails parallèles 54, 55 espacés d'une distance d sensiblement supérieure au diamètre dudit corps postérieur.

Lors de cet acheminement, et grâce à la présence des échancrures 52, 53, ce corps postérieur 9 est amené automatiquement à pivoter sur lui-même, de façon à venir se positionner dans une position stable dans laquelle les faces d'appui 50, 51 reposent sur les rails 54, 55.

Au terme de ce transfert, les corps postérieurs 9 sont ainsi délivrés avec une orientation sensiblement identique au niveau d'un premier poste d'assemblage adapté pour permettre d'introduire la bague de verrouillage 24 à l'intérieur dudit corps postérieur.

A cet effet, cette bague de verrouillage 24 est présentée en regard de la face antérieure du corps postérieur 9, jusqu'à amener les nervures 27 en butée contre l'épaulement 20. Il est à noter que cette mise en place est autorisée par l'élasticité des languettes 21 et à la forme de la face antérieure 22b des nervures 22 qui forme une rampe autorisant le franchissement desdites nervures.

Il est également à noter qu'une fois la bague de verrouillage 24 mise en place, celle-ci est bloquée en rotation relativement au corps postérieur 9 du fait du positionnement des nervures 27 dans les rainures 19.

L'ensemble corps postérieur 9 / bague de verrouillage 24 ainsi pré-assemblé est alors déplacé vers un deuxième poste d'assemblage.

Ce deuxième poste d'assemblage représenté schématiquement à la figure 13 comprend, en premier lieu, un bâti 56 dans lequel est ménagé un alésage 57 débouchant au niveau de la face supérieure dudit bâti par un lamage 57a de forme ovoïde conjuguée de celle du tronçon postérieur 8a du corps antérieur 8, permettant d'indexer la position dudit corps antérieur.

Ce poste d'assemblage comporte, en outre, une tige 58 adaptée pour s'étendre à l'intérieur du corps antérieur 8 positionné dans l'alésage 57, et dans le prolongement supérieur dudit corps antérieur, ladite tige servant de support à un ressort 42 monté autour de cette dernière de façon à reposer sur l'épaulement 10a des nervures 10 dudit corps antérieur.

Ce poste d'assemblage comprend, également des organes de maintien 59, de tout type connu en soi, adaptés pour positionner le corps postérieur 9 préalablement indexé en rotation, dans l'axe et au-dessus de la tige 58, lesdits organes de maintien étant associés à des moyens de déplacement en translation (non représentés) aptes à les déplacer selon un axe vertical.

Ce poste d'assemblage comprend, enfin, un outil de poussée 60 de forme tubulaire adaptée pour venir coiffer le tronçon postérieur du corps postérieur 9, le dit outil comprenant deux oreilles telles que 61 adaptées pour venir se loger chacune dans une échancrure 52, 53.

Un tel outil 60 permet en premier lieu de corriger d'éventuels écarts d'orientation, puis de déplacer verticalement le corps postérieur 9, sans risque d'une quelconque rotation de ce dernier, et enfin d'emmancher ledit corps postérieur sur le corps antérieur 8 par coopération du jonc de clipsage 12 et de la gorge 18. De plus, au cours de cette opération, le ressort 42 se trouve automatiquement comprimé entre les épaulements 10a des nervures 10 et l'épaulement 31. Enfin, un fois assemblés, les corps antérieur 8 et postérieur 9 se trouvent bloqués en rotation relative du fait du positionnement des ergots 13 dans les rainures 19.

Au terme de ces deux opérations, on obtient un ensemble de protection entièrement pré-assemblé, à l'intérieur duquel peut ensuite être introduite la seringue pré-remplie 1 tel qu'explicité ci-dessous.

Préalablement à cette introduction, l'embout à griffes 32 est positionné sur l'embout protège-aiguille 3 de la seringue 1, à ce stade dépourvue de tige de piston 5. Lors de ce positionnement, les pattes 34-37 de l'embout à griffes 32 viennent uniquement se positionner autour de l'embout protège-aiguille 3, sans risque d'enfoncer ce dernier et d'endommager l'aiguille 2 et/ou de détruire l'étanchéité.

La seringue 1 munie de l'embout à griffes 32 est ensuite introduite dans le corps postérieur 9 de l'étui protecteur 7 jusqu'à amener la collerette 1b à se bloquer entre les nervures 29 et l'épaulement 31. Il est à noter que cette introduction est autorisée par la faculté de se déformer des nervures 29 et à la forme de rampe de la face postérieure 29a de ces nervures 29 qui autorise le franchissement de ces dernières par la collerette 1b.

De plus, lors de cette introduction, l'embout à griffes 32 est amené à se resserrer sur l'embout protège-aiguille 3 lors du passage des bossages 40 de ce dernier au niveau de la rampe 15 du tronçon sécable 8b et du col 70, passage en outre facilité par la forme de rampe de la portion antérieure 40b desdits bossages.

Il est à noter, en outre, que tel que représenté à la figure 1, lors de ce resserrement, les griffes 38, 39 viennent s'ancrer dans l'embout protège-aiguille 3 en aval du cône en verre dont est muni traditionnellement le nez antérieur des seringues classiques 1, destiné à assurer l'étanchéité. Ainsi, tout risque d'enfoncement de l'embout protège-aiguille 3 et donc d'endommagement de l'aiguille 2 et/ou de destruction de l'étanchéité se trouve écarté.

Une fois cette étape réalisée, il convient de noter que l'embout à griffes 32 et l'étui protecteur 7 sont bloqués en rotation relative du fait de la coopération des crans respectifs 16, 41 de ces derniers. De plus, d'une part, l'embout protège-aiguille 3 est bloqué en rotation par rapport à l'embout à griffes 24 du fait de l'ancrage dans ce dernier des griffes 38, 39, et d'autre part, cet embout à griffes 32, l'étui protecteur 7 et la bague de verrouillage 24 sont également bloqués en rotation relative tel qu'explicité ci-dessus.

La dernière étape consiste enfin à solidariser de façon classique la tige de piston 5 au piston 4 puis à mettre en place le capuchon de protection 81. On obtient alors un dispositif d'injection prêt à l'emploi.

## Revendications

1. Dispositif d'injection comprenant une seringue (1) et un ensemble de protection de ladite seringue comportant un étui protecteur (7) tubulaire doté d'organes appui-doigts (23) destinés à la préhension dudit dispositif d'injection en vue de l'injection d'une dose de liquide notamment médicamenteux, ledit dispositif d'injection étant **caractérisé en ce que** les organes appui-doigts sont constitués d'une collerette (23), dotée d'une face supérieure et d'une face inférieure, comportant deux portions de contour symétriques (50, 51), dites de préhension, présentant des faces inférieures d'appui pour les doigts de l'utilisateur, et entre lesdites faces d'appui, deux portions de contour symétriques (52, 53) diamétralement opposées, dites d'indexation, comprenant dans la continuité de la face inférieure de ladite collerette, une face inférieure présentant un profil de forme convexe formé de deux pentes symétriques par rapport à un axe longitudinal.

2. Dispositif d'injection selon la revendication 1, **caractérisé en ce que** chaque portion d'indexation (52, 53) de la collerette (23) comporte une face inférieure présentant un profil courbe de forme arrondie convexe.

3. Dispositif d'injection selon l'une des revendications 1 ou 2, **caractérisé en ce que** chaque portion d'indexation (52, 53) de la collerette (23) présente la forme d'une ondulation définissant une échancrure de forme concave.

4. Dispositif d'injection selon la revendication 3, comprenant un capuchon de protection (81) apte à coiffer le tronçon postérieur de l'étui protecteur (7) de la seringue (1) de façon à venir en butée contre la collerette (23), **caractérisé en ce que** ledit capuchon de protection (8) comporte une paroi périphérique dotée d'un tronçon d'extrémité comprenant, pour chaque portion d'indexation (52, 53) de la collerette appui-doigts (23), une portion de paroi périphérique (82), dite d'assemblage, présentant un profil courbe de forme arrondie convexe complémentaire de la face supérieure de ladite portion d'indexation, adaptée pour s'emboîter dans cette dernière.

5. Dispositif d'injection selon la revendication 4, **caractérisé en ce que** le capuchon de protection (81) comporte au moins un organe interne (83, 84) de blocage en translation en saillie par rapport à la face interne de la paroi périphérique dudit capuchon, l'étui protecteur (7) de la seringue (1) comportant une paroi périphérique dont la face externe présente, en position conjuguée de chacun desdits organes de blocage en translation, un évidemment (80) apte à loger ce dernier.

6. Dispositif d'injection selon la revendication 5, **caractérisé en ce que** les organes de blocage en translation du capuchon de protection (81) consistent en deux ergots internes (83, 84) diamétralement opposés, chaque évidement du corps de seringue (1) consistant en une lumière (80) apte à loger un desdits ergots

## Patentansprüche

1. Injektionsvorrichtung, die eine Spritze (1) und eine Schutzanordnung für die Spritze aufweist, wobei die Schutzanordnung eine rohrförmige Schutzhülle (7) aufweist, die mit Fingerstützteilen (23) versehen ist, die in Hinblick auf die Injektion einer insbesondere medizinischen Flüssigkeitsdosis zum Greifen der Injektionsvorrichtung vorgesehen sind, wobei die Injektionsvorrichtung **dadurch gekennzeichnet ist, daß** die Fingerstützteile aus einem mit einer oberen Fläche und einer unteren Fläche versehenen Kragen (23) bestehen, der aufweist:
zwei symmetrische Konturabschnitte (50,51), sogenannte Greifabschnitte, die untere Stützflächen für die Finger des Benutzers aufweisen, und
zwischen den Stützflächen zwei diametral gegenüberliegende symmetrische Konturabschnitte (52,53), sogenannte Indexabschnitte, die in der Fortsetzung der unteren Fläche des Kragens eine untere Fläche aufweisen, die ein Profil konvexer Form aufweist, das aus zwei bezüglich einer Längsachse symmetrischen Schrägen gebildet ist.

2. Injektionsvorrichtung nach Anspruch 1, **dadurch gekennzeichnet, daß** jeder Indexabschnitt (52,53) des Kragens (23) eine untere Fläche aufweist, die ein Kurvenprofil einer runden konvexen Form aufweist.

3. Injektionsvorrichtung nach einem der Ansprüche 1 oder 2, **dadurch gekennzeichnet, daß** jeder Indexabschnitt (52,53) des Kragens (23) die Form einer Welle aufweist, die eine konkave Einbuchtung definiert.

4. Injektionsvorrichtung nach Anspruch 3, mit einer Schutzkappe (81), die so auf den hinteren Abschnitt der Schutzhülle (7) der Spritze (1) aufgesetzt werden kann, daß sie an dem Kragen (23) anliegt, **dadurch gekennzeichnet, daß** die Schutzkappe (81) eine Umfangswand aufweist, die mit einem Endabschnitt versehen ist, der für jeden Indexabschnitt (52,53) des Fingerstütz-Kragens (23) einen Umfangswandabschnitt (82), genannt Montage-Umfangswandabschnitt, aufweist, der ein Kurvenprofil einer runden konvexen Form aufweist, die komplementär zur oberen Fläche des Indexabschnitts ist, um sich in letzterer einfügen zu lassen.

5. Injektionsvorrichtung nach Anspruch 4, **dadurch gekennzeichnet, daß** die Schutzkappe (81) mindestens eine innere Translationsblockierungseinrichtung (83,84) aufweist, die bezüglich der Innenfläche der Umfangswand der Kappe vorspringt, wobei die Schutzhülle (7) der Spritze (1) eine Umfangswand aufweist, deren Außenfläche an einer entsprechenden Gegenposition zu jeweils einer Translationsblockierungseinrichtung eine Aussparung (80) aufweist, um letzte aufzunehmen.

6. Injektionsvorrichtung nach Anspruch 5, **dadurch gekennzeichnet, daß** die Translationsblockierungseinrichtungen der Schutzkappe (81) aus zwei diametral gegenüberliegenden inneren Nocken (83.84) bestehen, wobei jede Aussparung des Spritzenkörpers (1) aus einem Langloch (80) besteht, das einen der Nocken aufnehmen kann.

## Claims

1. An injection device comprising a syringe (1) and an assembly to protect said syringe comprising a tubular protective case (7) provided with finger-support parts (23) intended for gripping said injection device for the injection of a dose of liquid, particularly of a medicinal liquid, **characterised in that** the finger-support parts consist of a flange (23), provided with a top face and a bottom face, comprising two so-called gripping portions (50, 51) of symmetrical contour, having bottom support surfaces for the user's fingers, and between said support surfaces, two diametrically opposed, so-called indexing portions (52, 53) of symmetrical contour, comprising in the continuity of the bottom face of said flange, a bottom face having a convex profile formed by two symmetrical slopes with respect to a longitudinal axis.

2. An injection device according to claim 1, **characterised in that** each indexing portion (52, 53) of the flange (23) comprises a bottom face having a convex rounded curved profile.

3. An injection device according to any of claims 1 or 2, **characterised in that** each indexing portion (52, 53) of the flange (23) has an undulating shape defining a concave-shaped scalloping.

4. An injection device according to claim 3, comprising a protective cap (81) adapted to cover the rear section of the protective case (7) of the syringe (1) so as to abut against the flange (23), **characterised in that** said protective cap (8) comprises a peripheral wall provided with an end section comprising, for each indexing portion (52, 53) of the finger-support flange (23) a so-called (82) peripheral wall assembly portion, having a convex rounded curved profile complementary to the top face of said indexing portion, adapted to fit into said portion.

5. An injection device according to claim 4, **characterised in that** the protective cap (81) comprises at least one internal translation blocking part (83, 84) projecting from the inner face of the peripheral wall of said cap, the protective case (7) of the syringe (1) comprising a peripheral wall, the outer face of which comprises, in the combined position of each of said translation blocking parts, a recess (80) adapted to house said part.

6. An injection device according to claim 5, **characterised in that** the translation blocking parts of the protective cap (81) consist of two diametrically opposed internal pins (83, 84), each recess of the syringe body (1) consisting of a slot (80) adapted to house one of said pins.
